# EUROPEAN PATENT SPECIFICATION

(11) **EP 0 386 146 B1**
(45) Date of publication and mention of the grant of the patent: **28.04.1993**
(21) Application number: 89900703.3
(22) Date of filing: 03.11.1988
(51) Int. Cl.: A61K 43/00

(54) **COMPOSITION AND METHOD OF USE FOR LIPOSOME ENCAPSULATED COMPOUNDS FOR NEUTRON CAPTURE TUMOR THERAPY**
ZUSAMMENSETZUNG UND VERFAHREN ZUR ANWENDUNG VON IN LIPOSOMEN EINGESCHLOSSENEN VERBINDUNGEN IN DER NEUTRONENEINFANGTHERAPIE VON TUMOREN
COMPOSITION ET PROCEDE D'UTILISATION DE COMPOSES ENCAPSULES DANS DES LIPOSOMES POUR UNE THERAPIE DE TUMEURS PAR CAPTURE DE NEUTRONS

(30) Priority: 04.11.1987 US 116764
(43) Date of publication of application: 12.09.1990
(73) Proprietor: VESTAR, INC., San Dimas, CA 91773 (US)
(72) Inventor: FUJII, Gary, Torrance, CA 90504 (US); SCHMIDT, Paul, Gardner, San Marino, CA 91108 (US); GAMBLE, Ronald, Carl, Alta Dena, CA 91001 (US)
(74) Representative: Goldin, Douglas Michael
(86) International application number: US8803820
(87) International publication number: WO8904176

(56) References cited:
- US-A- 4 086 330
- US-A- 4 588 578
- US-A- 4 674 480

## Description

### 1. Field of the Invention

This invention generally relates to a composition for treating tumors, and more particularly to a composition of treating tumors using liposome encapsulated compounds in neutron capture therapy.

### 2. Background of the Invention

Neutron capture therapy is an attractive method for cancer therapy, specifically the treatment of malignant tumors. The generalized reaction involves capture of a thermalized neutron (usually from a nuclear reactor with special moderators and ports) by an appropriate nucleus having a large neutron capture cross section. The subsequent decay emits energetic particles (alpha particles) which can kill nearby tumor cells. Boron-10, for example, has such an appropriate nucleus and has particularly advantageous properties for this scheme. The boron-10/thermal neutron capture reaction is (asterisk indicating an unstable intermediate state of the Boron nucleus):

10_{B} + ¹n ---> [¹¹B]* ---> ⁷Li (0.87 Mev.) + ⁴He (1.52 Mev)

In order for this therapy to be effective, sufficient ¹⁰B must be localized in a tumor to generate the required density of particles. This level has been variously estimated to be approximately 10-50 µg ¹⁰B/gm tumor. Furthermore, the concentration of ¹⁰B in normal tissue and blood should be limited and preferably less than the concentration in tumor in order to minimize damage to healthy cells and blood vessels. H. Hatanaka (1986) Boron-Neutron Capture Therapy for Tumors; Nishimura Co., Ltd. p. 1-16.

Large numbers of boron-containing compounds have been tested for their ability to satisfy the above criteria. With few exceptions all have failed as not enough boron has localized in the tumor and the concentration in the blood has been too high for effective neutron capture therapy. Human clinical trials with Na₂B₁₂H₁₁SH in Japan have shown some promise, but only for a limited group of brain tumors. (gliomas) Id. 16-26.

Neutron capture therapy would be greatly expanded in usefulness if a generalized method for delivering high concentrations of ¹⁰B to tumors were available. It would further be useful if more ¹⁰B collected in tumor than in the blood. Recently, it has become possible to deliver drugs and other compounds selectively to tumors using liposomes of a particular composition and structure. See, for example, co-pending Vestar, Inc. patent application EP-A-0 182 131 entitled "Method of Targeting Tumors in Humans" which describes incorporation of radioactive agents at levels a million fold less than what is required for successful neutron capture therapy. Incorporation of compounds with higher osmolarity inside liposomes than outside, as is necessary for effective neutron capture therapy, has heretofore never been achieved and has been considered to be an unstable condition since the extra osmotic pressure should lead to breakage of the liposomes and/or leakage of the contents. Successful neutron capture therapy with liposomes depends on incorporating the highest concentration of ¹⁰B possible without substantially altering the liposome's favorable biodistribution characteristics. That can be accomplished by using compounds with the highest number of boron atoms practicable and by incorporating boron into liposomes at the highest possible concentration consistent with liposomes that are stable and target to tumors. It was found in the present invention that hyperosmotic solutions could be stably encapsulated while still maintaining good animal biodistribution performance. Thus, the objective of at least 10 µg ¹⁰B per gram of tumor tissue could be met (assuming use of >90% ¹⁰B enriched material). The present invention thus offers a method employing specially formulated liposomes, which encapsulate unexpectedly high concentrations of boron-containing compounds, and which collect in tumor tissue after intravenous injection or direct infusion into the artery that supplies the tumor. After a time to enable sufficient accumulation of boron-containing compounds in the tumor tissue, the subject can be subjected to a source that emits neutrons effective for neutron capture therapy.

### Summary Of The Invention

It is an object of the invention to provide composition suitable for use in a method for delivering therapeutically useful concentrations of boron-containing compounds to tumors for use in neutron capture tumor therapy.

It is a further object to provide a liposome formulation, encapsulating boron-containing compounds, that has the properties of retaining concentrations of said boron compounds inside the liposomes without significant breakage.

It is a further object of the invention to provide use of a composition in the manufacture of a medicament for cancer therapy through use of liposomal encapsulated boron-containing compounds, the means to deliver at least 10 micrograms ¹⁰B per gram of tumor tissue to animal and human tumors, while minimizing the concentration in the blood.

### Detailed Description Of The Invention

Liposomes are microscopic structures consisting in part of phospholipids. Methods for forming these liposomes are, by now, well known in the art and any such methods can be employed in the context of the present invention. Phospholipids are composed of 2 fatty acid chains condensed with glycerol with an additional substitution of a phosphate ester head group. We have found that by incorporating certain phospholipid molecules, a liposome is obtained which is stable in vivo. It is known that phase transition points are a function of hydrocarbon chain length, C. Lanford, The Hydrophobic Effect, 2d Ed (1980). Certain phospholipid molecules exhibit phase transitions at relatively high temperatures (greater than 37°C) and we have found that use of these phospholipids in the compositions described herein provide liposomes with improved stability in vivo. When hydrated, phospholipids form into bilayer structures with their fatty acid hydrocarbon tails pointed inward and the polar head groups outward. A hydrated phospholipid suspension, when agitated, forms multilamellar vesicles (MLV) like tiny onions, with water separating many bilayers. Application of a shearing force such as sonication to an MLV suspension produces small single bilayer vesicles (SUV) of a size range generally 30-200 nm in average diameter. Other lipids may be combined with phospholipids to produce liposomes having particular properties. Specifically sterols such as cholesterol help stabilize the bilayer toward leakage and destruction in blood plasma. A stable liposome may be obtained by incorporating 5-50% cholesterol by weight of phospholipid into the liposome. Charged lipids or lipids with specific ligand functions may also be included.

SUV comprised of distearoylphosphatidylcholine (DSPC) and cholesterol are particularly advantageous for delivery of radioactive compounds to tumors.

There are non-radioactive isotopes other than boron-10 that do show a large capture cross-section for thermal neutrons suitable for the present invention, namely: ³He, ¹⁴⁹Sm, ²³⁵U, ⁶Li, ¹¹³Cd, ^{155,157}Gd. Except for lithium and uranium however, the other listed elements also produce gamma radiation after absorbing a thermal neutron. The resulting gamma rays are not confined to the target cells, and therefore are less desirable for use with the present invention, although certainly capable of being used therein.

Boron compounds to be used can have two or more atoms of boron per molecule, but preferably contain at least 10 atoms of boron per molecule. The isotopic content of the boron can range from natural abundance 19.78% ¹⁰B to greater than 95% ¹⁰B for a highly enriched compound. Natural abundance material is useful for test studies of encapsulation, biodistribution and stability. Highly enriched material is advantageous for therapy where the maximum practicable concentration of ¹⁰B is required.

Preferable boron-containing compounds are highly water soluble, have small or no charge at physiological pH, are relatively impermeable to phospholipid bilayers of liposomes, and are not toxic or have low toxicity to the therapy subject. Examples are Na₂B₁₀H₁₀, Na₂B₁₂H₁₂, Na₂B₁₂H₁₁SH, and Na₂B₁₀O₁₆. A concentration of at least 250 mM inside of the liposome is necessary for a boron compound having at least 10 boron atoms per molecule. Of course, with a lower amount of boron atoms per molecule, a higher concentration is required.

In one preferred embodiment liposomes are prepared from a mixture of DSPC and cholesterol (1:1 molar ratio). The dried lipid film is hydrated with an aqueous solution of Na₂B₁₀H₁₀. Shear force is applied to the solution by sonication or other suitable homogenizing technique to produce small unilamellar vesicles (SUV) whose average diameter is less than 200 nm and preferably 30-100 nm. The non-encapsulated Na₂B₁₀H₁₀ is separated from the liposomes by gel filtration chromatography using a Sephadex G-25-80® column equilibrated with phosphate buffered normal saline, or with lactose at an osmolarity approximately equal to physiological. The resulting liposome solution is stable to leakage of the material inside, such that less than 10% of the boron material leaks out over a period of 3.5 months (Tables 2 and 3).

The cholesterol-DSPC liposomes with 250 mM Na₂B₁₀H₁₀ and ¹¹¹In-EDTA as a radioactive tracer inside are injected into the tail vein of Balb/c mice. The animals have previously been implanted subcutaneously with EMT6 tumor cells in the right flank. At various time points the animals are sacrificed and their blood, tumor, liver and spleen are tested for concentrations of the ¹¹¹In compound by measuring gamma radiation levels. Insofar as the In-EDTA complex behaves like B₁₀H₁₀²^{⁻}, the radioactive tracer represents the biodistribution of the boron compound. The results show that boron 10 can be delivered to tumors at levels sufficient to give therapeutic value in neutron capture therapy.

The invention extends to other boron compounds where the number of boron atoms per molecule is at least two, preferably 10 and to polymeric boron compounds so as to maximize the boron concentration inside the liposome. The invention includes performing neutron capture tumor therapy by administering boron-containing liposomes and thereafter, subjecting the patient to a source that emits neutrons. Such a source was described for example in U.S. Patent No. 4,516,535 issued to Russell, Jr. et al.

### Preparation of Na₂B₁₀H₁₀ in water (normal isotopic abundance).

The Na₂B₁₀H₁₀ was prepared from the bis-triethylammonium salt of B₁₀H₁₀ = by methods well known in the art.

The bis-triethylammonium salt of B₁₀H₁₀ = was synthesized by the procedure set forth in Hawthorne, M.F.; Pilling, R.L., Inorg. Syn, 1967, 9, 16.

### Preparation Of Liposome Encapsulated Na₂B₁₀H₁₀

To a 15 ml glass tube was added 166 mg DSPC (Avanti Polar Lipids, Birmingham, Alabama) and 84 mg cholesterol (Calbiochem, San Diego, California) (molar ratio 1:1) in 5.0 ml CHCl₃/methanol (75:25 v/v). The solution was evaporated with a stream of nitrogen to form a film on the inside of the tube. The tube was placed under vacuum for at least 12 hours. To this tube was added 5.0 ml of a 250 mM solution of Na₂B₁₀H₁₀ in distilled water. The osmolarity of the 250 mM Na₂B₁₀H₁₀ solution was separately measured using an osmometer (model OW2, Advanced Instruments, Needham Heights, Mass.) and found to be 730 mOsm/lit. The hydrated lipid sample was sonicated using a Sonics and Materials Vibracell probe sonicator with a microtip operated at the maximum power setting for the microtip. The solution was maintained at 65°C and under N₂ atmosphere and sonicated for 15 minutes. The sample was then allowed to cool to room temperature. The process was repeated for a second sample.

The processed liposome samples were separately applied to Sephadex G-25-80® columns (10 x 1 cm) equilibrated with 5 mM phosphate pH 7.5 in 0.9% saline (PBS) or 90 mg/ml lactose. Both of these solutions in the columns were at 295 mOsm/lit osmolarity. The liposomes were eluted by vacuum, in the process exchanging their outside Na₂B₁₀H₁₀ for PBS or lactose. The liposome containing fraction was collected from the column.

### Size Analysis of Liposome Encapsulated Na₂B₁₀H₁₀

The size of the liposomes was determined by dynamic light scattering using a Nicomp model 270 laser light scattering unit. A volume of 20 microliters of the sample were diluted into 750 microliters phosphate buffered saline (PBS) for the measurement. Results for the Gaussian distribution mode are shown below in Table 1.

**TABLE 1**

| Sample Stored in | Mean Diameter (volume weighed) |
|---|---|
| PBS | 69.8 nm |
| Lactose | 75.3 nm |

### Encapsulated Concentration of Na₂B₁₀H₁₀

The encapsulated concentration of Na₂B₁₀H₁₀ was gauged by measuring the total decahydrodecaborate concentration by a colorimetric method in each sample and then in the effluent after ultrafiltration to correct for material outside the liposomes.

The colorimetric method is described in Hawthorne, M.F., Olsen, F. P. J.Am.Chem. Soc., 1965, 87, 2366,.

This colorimetric method depends upon the rapid and quantitative formation of an intensely colored blue azo dye (λₘₐₓ5200 Δ ε = 2 x 10⁴) produced by coupling benzene diazonium ion with the 1-position of the B₁₀H₁₀²⁻ ion in acetonitrile solution in the presence of trifluoroacetic acid. This method can quantitatively determine B₁₀H₁₀²⁻ at concentrations as low as 1 x 10⁻⁵ M. The results are shown below in Table 2.

**TABLE 2**

| Liposome Sample in | Total | Na₂B₁₀H₁₀ (mM) | |
|---|---|---|---|
| | | Outside | Inside |
| PBS | 4.8 | 0.35 | 4.45 |
| Lactose | 3.5 | 0.18 | 3.32 |

Very little of the decahydrodecaborate is outside the liposome initially (5-8%).

### Encapsulated Concentration of Na₂B₁₀H₁₀ (after 3.5 months)

The samples were stored at room temperature (21-23°C) for 3.5 months and then retested.

**TABLE 3**

| Liposome Sample in | Total | Na₂B₁₀H₁₀ (mM) | |
|---|---|---|---|
| | | Outside | Inside |
| PBS | 4.8 | 0.63 | 4.17 |
| Lactose | 3.5 | 0.11 | 3.39 |

After 3.5 months the liposomes stored in PBS have leaked only about 6% more boron compound from the inside to outside, while those stored in lactose have lost no boron within the experimental error of the measurement. Thus the liposomes of the invention can maintain for months a solution of 730 mOsm/lit. decahydrodecaborate inside while the outside solution is only 295 mOsm/lit.

### Biodistribution of Boron encapsulated Liposomes in Mice Using an ¹¹¹In chelate liposome label

Liposome samples were prepared as discussed above except that the ionophore A23187 was included in the lipid at a level of 10⁻³ of the weight of the phospholipid. The hydrating solution [2.5 ml] was 250mM M Na₂B₁₀H₁₀ and 1 mM EDTA. The outside solution of the sonicated liposomes was exchanged for PBS by vacuum elution from Sephadex G-25-80® as before. These liposomes had a mean diameter of 57.9 nm. Lipid analysis showed 14.3 mg/ml DSPC and 7.67 mg/ml cholesterol. A sample of the exchanged liposomes was incubated with 50 ul radioactive ¹¹¹InCl₃ and 5 mM sodium citrate at 80°C for 30 minutes. ¹¹¹In⁺³ is transported by A23187 to the EDTA inside the liposome in this procedure. Tests of In concentration in the total sample and outside after loading showed a loading efficiency of 93%, that is, 93% of the added ¹¹¹In was inside the liposomes as the In-EDTA complex. A similar preparation using the ultrafiltration step and colorimetric assay for Na₂B₁₀H₁₀ showed that less than 1% of the Na₂B₁₀H₁₀ had leaked out of the liposomes during the 30 min. incubation at 80°.

¹¹¹In chelated to EDTA is used as a radioactive tracer in this experiment since direct measurement of the B₁₀H₁₀⁻² species in animal tissues proved to be impossible by known methods. The In-EDTA complex is exceedingly stable so that the radioactive ion does not dissociate to any appreciable extent in the body. The complex is negatively charged and highly water soluble as is the B₁₀H₁₀⁻² ion. Thus, the In-EDTA and B₁₀H₁₀⁻² are expected to have very similar pharmacokinetics in mouse biodistribution studies. Therefore, the percent injected dose per gram tissue measured from the radioactivity of ¹¹¹In gamma emission should correspond closely to the distribution of B₁₀H₁₀⁻².

The ¹¹¹In-EDTA loaded liposomes still containing 250 mM Na₂B₁₀H₁₀ (inside concentration) were injected into the tail vein of Balb/c mice having an 8 day old implanted EMT6 tumor in the flank. A volume of 200 microliters of the liposome sample was injected in each mouse. Mice were sacrificed in groups of 5 at 24, 48, and 72 hours after injection. Tissues were dissected, weighed and analyzed for radioactivity in a gamma counter. The results, expressed as the average percent ¹¹¹In injected dose per gram (% ID/gram) tissue are listed below:

**TABLE 4**

| Tissue | Time Post Injection (hrs) | | |
|---|---|---|---|
| | 24 | 48 | 72 |
| Blood | 7.8 | 0.5 | 0.08 |
| Tumor | 10.3 | 3.8 | 2.2 |
| Liver | 11.4 | 3.8 | 2.3 |
| Spleen | 14.7 | 7.9 | 6.4 |

At 24 hours the tumor level is greater than the blood, optimal for neutron capture therapy.

As set forth in Table 4, after 24 hours the In uptake in the tumor is 10.3% injected dose per gram (ID/gram). We can therefore estimate the amount of ¹⁰B deliverable to a tumor at the 24 hour level as likewise 10.3% injected dose per gram. The injected dose was 200 microliters of a liposome sample of 21.97 mg/ml lipid concentration. Liposomes of the kind used here encapsulate at least 1.0 microliter of solution per mg. lipid. That means 21.97 microliters or 2.2% are entrapped per ml of solution in the sample used. The injected dose of 100 microliters contained 0.2 ml x .022 x 250 micromoles/ml B₁₀H₁₀⁻² or 1.10 micromoles B₁₀H₁₀⁻². If the isotopic enrichment were 90% ¹⁰B or greater, the total ¹⁰B injected would be 1.10 micromoles x 90 micrograms ¹⁰B micromole B₁₀H₁₀⁻² = 99.0 micrograms ¹⁰B. For a tumor level of 10.3 %ID/gm the tumor concentration would then be 10.2 micrograms ¹⁰B per gram tumor which is within the range considered to be necessary for successful neutron capture therapy.

Although this invention has been described with reference to particular applications, the principles involved are susceptible of other applications which will be apparent to those skilled in the art. The invention is accordingly to be limited only by the scope of the appended claims.

## Claims

1. A neutron capture therapy composition comprising stable unilamellar liposomes having an average diameter of from 30 to 200 nm encapsulating a compound at a concentration of greater than 250 mM, the compound containing an element which has a large neutron cross-section and is capable of forming an isotope which emits an alpha particle when bombarded with neutrons.

2. A composition as claimed in claim 1 in which the compound contains the elements ¹⁰B, ²³⁵U or ⁶Li.

3. A composition as claimed in claim 1 or 2 in which the liposomes are comprised of phospholipids having a phase transition temperature greater than 37°C.

4. A composition as claimed in any one of the preceding claims in which the compound is a boron compound.

5. A composition as claimed in claim 4 in which the boron compound contains at least 10 atoms of boron per molecule.

6. A composition as claimed in claim 5 in which the boron compound is selected from Na₂B₁₀H₁₀, Na₂B₁₂H₁₂, Na₂B₁₂H₁₁SH, or Na₂B₁₀O₁₆.

7. A composition as claimed in any one of claims 4, 5 or 6 in which the boron compound is water soluble and has little or no charge at physiological pH.

8. A composition as claimed in any one of claims 4 to 7 in which the boron compound is encapsulated in a concentration sufficient to accumulate said isotope in a tumor at a concentration of 10 micrograms per gram to 50 micrograms per gram tumor.

9. A method for the preparation of a composition as defined in any one of claims 1 to 8 comprising forming unilamellar liposomes having an average diameter of from 30 to 200 nm in a aqueous solution having a concentration of at least 250 mM of a compound having an element which has a large neutron capture cross section, the element being capable of forming an isotope which emits an alpha particle when bombarded with neutrons; and exchanging the outside solution of the liposomes to form a dispersion of stable liposomes having an internal concentration of the compound which is of higher osmolarity inside the liposomes than outside.

10. The use of a composition as defined in any one of claims 1 to 8 or as produced in claim 9 in the manufacture of a medicament for use in the treatment of a tumor.

## Patentansprüche

1. Zusammensetzung für die Neutroneneinfang-Therapie, umfassend stabile unilamellare Liposome mit einem durchschnittlichen Durchmesser von 30 bis 200 nm, die eine Verbindung in einer Konzentration von größer als 250 mM einkapseln, wobei die Verbindung ein Element enthält, das einen großen Wirkungsquerschnitt für Neutronen besitzt und dazu fähig ist, ein Isotop zu bilden, das ein Alpha-Teilchen emittiert, wenn es mit Neutronen beschossen wird.

2. Zusammensetzung nach Anspruch 1, dadurch gekennzeichnet, daß die Verbindung die Elemente ¹⁰B, ²³⁵U oder ⁶Li enthält.

3. Zusammensetzung nach Anspruch 1 oder 2, dadurch gekennzeichnet, daß die Liposome aus Phospholipiden mit einer Phasenumwandlungstemperatur von größer als 37°C aufgebaut sind.

4. Zusammensetzung nach einem der vorhergehenden Ansprüche, dadurch gekennzeichnet, daß die Verbindung eine Borverbindung ist.

5. Zusammensetzung nach Anspruch 4, dadurch gekennzeichnet, daß die Borverbindung mindestens 10 Boratome pro Molekül enthält.

6. Zusammensetzung nach Anspruch 5, dadurch gekennzeichnet, daß die Borverbindung ausgewählt ist aus Na₂B₁₀H₁₀, Na₂B₁₂H₁₂, Na₂B₁₂H₁₁SH oder Na₂B₁₀O₁₆.

7. Zusammensetzung nach einem der Ansprüche 4, 5 oder 6, dadurch gekennzeichnet, daß die Borverbindung wasserlöslich ist und bei physiologischem pH-Wert eine geringe oder keine Ladung besitzt.

8. Zusammensetzung nach einem der Ansprüche 4 bis 7, dadurch gekennzeichnet, daß die Borverbindung in einer Konzentration eingekapselt ist, die ausreicht, um das Isotop in einem Tumor mit einer Konzentration von 10 bis 50 µg/g Tumor anzureichern.

9. Verfahren zur Herstellung einer Zusammensetzung nach einem der Ansprüche 1 bis 8, dadurch gekennzeichnet, daß man in einer wässerigen Lösung unilamellare Liposome mit einem durchschnittlichen Durchmesser von 30 bis 200 nm bildet, wobei die wässerige Lösung eine Verbindung in einer Konzentration von mindestens 250 mM enthält, aie ein Element mit einem großen Wirkungsquerschnitt für Neutronen besitzt, wobei das Element dazu fähig ist, ein Isotop zu bilden, das ein Alpha-Teilchen emittiert, wenn es mit Neutronen beschossen wird; und die außenseitige Lösung der Liposomen austauscht, um eine Dispersion von stabilen Liposomen zu bilden, die eine innere Konzentration der Verbindung besitzen, die innerhalb der Liposome eine höhere Osmolarität als außerhalb aufweist.

10. Verwendung einer Zusammensetzung nach einem der Ansprüche 1 bis 8 oder einer nach Anspruch 9 hergestellten Zusammensetzung zur Herstellung eines Arzneimittels zur Verwendung in der Tumorbehandlung.

## Revendications

1. Composition pour thérapie par capture de neutrons comprenant des liposomes unilamellaires stables ayant un diamètre moyen de 30 à 200 nm encapsulant un composé à une concentration supérieure à 250 mM, le composé contenant un élément qui présente une importante section efficace aux neutrons et qui est capable de former un isotope qui émet une particule alpha lorsqu'on le bombarde avec des neutrons.

2. Composition selon la revendication 1 dans laquelle le composé contient les éléments ¹⁰B, ²³⁵U ou ⁶Li.

3. Composition selon la revendication 1 ou 2 dans laquelle les liposomes comprennent des phospholipides ayant une température de transition de phases supérieure à 37°C.

4. Composition selon l'une quelconque des revendications précédentes dans laquelle le composé est un composé de bore.

5. Composition selon la revendication 4 dans laquelle le composé de bore contient au moins 10 atomes de bore par molécule.

6. Composition selon la revendication 5 dans laquelle le composé de bore est choisi parmi Na₂B₁₀H₁₀, Na₂B₁₂H₁₂, Na₂B₁₂H₁₁SH ou Na₂B₁₀O₁₆.

7. Composition selon l'une quelconque des revendications 4, 5 ou 6 dans laquelle le composé de bore est soluble dans l'eau et présente peu ou pas de charge au pH physiologique.

8. Composition selonl'une quelconque des revendications 4 à 7 dans laquelle le composé de bore est encapsulé à une concentration suffisante pour accumuler ledit isotope dans une tumeur à une concentration de 10 microgrammes par gramme à 50 microgrammes par gramme de tumeur.

9. Procédé de préparation d'une composition telle que définie dans l'une quelconque des revendications 1 à 8 comprenant les étapes consistant à former des liposomes unilamellaires ayant un diamètre moyen de 30 à 200 nm dans une solution aqueuse ayant une concentration d'au moins 250 mM d'un composé ayant un élément présentant une importante section efficace de capture neutronique, l'élément étant capable de former un isotope qui émet une particule alpha lorsqu'on le bombarde avec des neutrons ; et à échanger la solution externe de liposomes pour former une dispersion de liposomes stables ayant une concentration interne du composé dont l'osmolarité à l'intérieur des liposomes est supérieure à celle à l'extérieur.

10. Utilisation d'une composition selon l'une quelconque des revendications 1 à 8 ou telle que produite selon la revendication 9 dans la préparation d'un médicament destiné à être utilisé dans le traitement d'une tumeur.
